# EUROPEAN PATENT APPLICATION

(11) **EP 0 890 350 A1**
(43) Date of publication of application: **13.01.1999**
(21) Application number: 97111501.9
(22) Date of filing: 08.07.1997
(51) Int. Cl.: A61F 13/15

(54) **Disposable absorbent articles with clothlike feel backsheet having zoned breathability and process for making such backsheets**

(71) Applicant: THE PROCTER & GAMBLE COMPANY, Cincinnati, Ohio 45202 (US)
(72) Inventor: Isele, Olaf, (NMN), 61389 Schmitten-Oberreifenberg (DE); Beruda, Holger, Dr. (NMN), 65779 Kelkheim-Ruppertshain (DE)
(74) Representative: Canonici, Jean-Jacques

(57) **Abstract**

A disposable absorbent article comprising a breathable polymeric film at least partially combined with a fibrous material to a laminated for being used as backsheet material with zones having different breathability.

## Description

### Field of the Invention

The present invention relates to disposable absorbent articles such as baby diapers, incontinence articles, sanitary towels and the like, and in particular to baby diapers and adult incontinence articles having backsheets with a clothlike feel and particular breathability.

### Background of the Invention

Disposable, absorbent articles such as diapers, incontinence articles, sanitary towels, training pants and the like are well known in the art. Typically, disposable absorbent articles comprise a liquid pervious topsheet that faces the wearer's body, a liquid impervious backsheet that faces the wearer's clothing, an absorbent core interposed between the liquid pervious topsheet and the backsheet, and means to keep the core in fixed relation to the wearer's body.

In order to receive the body exudates such as urine, faeces or menstrual fluids, the article has to cover certain parts of the wearer's body. Generally, current articles cover even larger parts of the wearer's body to allow for adequate storage of the exudates. Whilst this coverage is an essential element of the functionality of the article, the article also can - beyond impacting on the comfort of the wearer - induce negative impact on the skin, such as by exerting pressure on the skin, or by creating occlusion for certain parts of the skin, thereby potentially inducing over-hydration of the skin, in particular under conditions where the wearer has some tendency for sweating.

Numerous attempts have been disclosed aiming at improving on the skin condition of the wearer by allowing the over-hydrated skin to dehydrate to an acceptable level by allowing either air to reach the skin thus minimising potential occlusion effects, and/or by water vapour being removed from the surface of the skin. Generally, such mechanisms are referred to as "breathability" or "vapour or moisture permeability".

A number of such applications aim at feminine hygiene products, such as catamenial products or so-called "panty-liner" as described in EP-A-0.104.906; EP-A-0.171.041; EP-A-0.710.471. Such products generally have relatively low fluid storage capacity when compared for example to baby diapers or adult incontinence products, often being designed for theoretical capacities significantly exceeding the ones for the feminine hygiene products.

Breathable materials for use in various articles can be various kinds of webs, such as films which were rendered air/vapour permeable by aperturing as described in U.S.-A-5.628.737, or by exploiting the "microporosity" property as described in EP-A-0.238.200; EP-A-0.288.021; EP-A-0.352.802; EP-A-0.515.501; U.S.-A-4.713.068, whereby small voids are created within the film similar to very small cracks.

Also known is the combination of such films with non-woven materials, such as described in WO 97/15442, wherein polymeric films containing filler materials such as calcium carbonate are extrusion coated onto a non-woven web having a wider width than the film, whereby the breathability of the film is created by subsequent interdigitating stretching.

However, prior art did not recognise the benefits that arise when using a backsheet material comprising a breathable film material having different degrees of breathability in different zones thereof, in particular when at least one of these regions further comprises a fibrous material providing an improved hand and feel.

Hence, it is an object of the present invention to provide an absorbent article comprising an absorbent core defining a core region, a chassis region surrounding said core region, a backsheet material comprising at least in the core region a laminate comprising a vapour or gas permeable film or film-like material, and further comprising a fibrous layer; whereby at least one polymeric film layer of the core backsheet and the chassis backsheet is unitary over both regions, and whereby the backsheet material in the core region has a lower breathability as expressed in MVTR values than the backsheet material in the chassis region.

It is a further object of the present invention to provide a process for making such a backsheet for being used in such an article by combining a filled poly film with a narrower non-woven strip, then interdigitatingly activating the laminate either with even activation conditions throughout the full width, thereby creating uneven breathability in the laminated and pure film zones, or with uneven activation conditions, thereby enhancing the breathability difference between the two zones.

It is an even further object to provide materials as made by this process.

### Summary of the Invention

A disposable absorbent article comprising a breathable polymeric film at east partially combined with a fibrous material to a laminated for being used as backsheet material with zones having different breathability

### Brief Description of the Drawings

Figure 1 is schematically showing a taped baby diaper as an example for an absorbent article.
Figure 2 shows a schematic diagram of an apparatus which can be used in a preferred execution of the pressure application stretching equipment.
Figure 3 shows schematically a cross-section through the apparatus.

### Detailed Description

### Absorbent Articles - general

As used herein, the term "absorbent articles" refers to devices which absorb and contain body exudates, and, more specifically, refers to devices which are placed against or in proximity to the body of the wearer to absorb and contain the various exudates discharged from the body.

The term "disposable" is used herein to describe absorbent articles which are not intended to be laundered or otherwise restored or reused as an absorbent article (i.e., they are intended to be discarded after use and, preferably, to be recycled, composted or otherwise disposed of in an environmentally compatible manner).

Within the context of the present invention absorbent article comprises:
a) - an absorbent core (which may consist of sub-structures and/or wrap materials), including on the side oriented towards the wearer a topsheet, which forms the inner surface and which - at least in certain regions thereof - allows the exudates to penetrate through, and including on the opposite side a backsheet which forms the outer surface of the article and which separates the absorbent core from the outside, such as the clothing of the wearer.
b) - chassis elements comprising features like closure elements or elastication to maintain the article on the wearer. Also comprising a topsheet which forms the inner surface and a backsheet. The backsheet and the topsheet materials of the absorbent core can be unitary with respective materials in the chassis regions, i.e. the backsheet can cover the absorbent core and the same material or sheet may extend into the chassis region, thereby, for example, covering features like the leg elastics or the like.

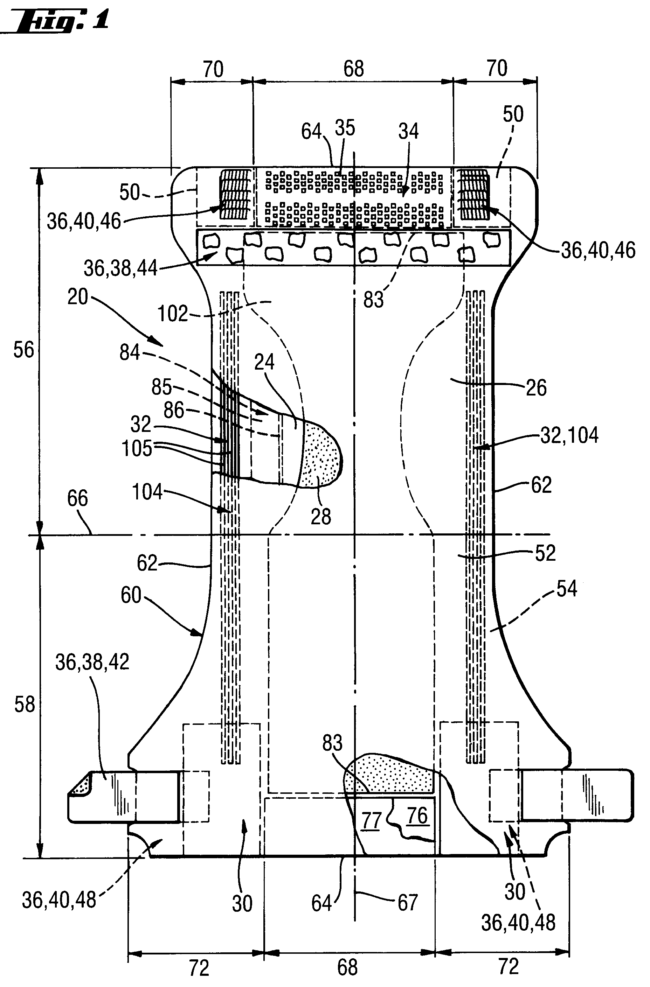

Figure 1 is a plan view of an embodiment of an absorbent article of the invention which is a diaper.

The diaper 20 is shown in Figure 1 in its flat-out, uncontracted state (i.e. with elastic induced contraction pulled out except in the side panels wherein the elastic is left in its relaxed condition) with portions of the structure being cut-away to more clearly show the construction of the diaper 20 and with the portion of the diaper 20 which faces away from the wearer, the outer surface 52, facing the viewer. As shown in Figure 1, the diaper 20 comprises a liquid pervious topsheet 24, a liquid impervious backsheet 26 joined with the topsheet 24, and an absorbent core 28 positioned between the topsheet 24 and the backsheet 26; elasticised side panels 30; elasticised leg cuffs 32; an elastic waist feature 34; and a closure system comprising a dual tension fastening system generally multiply designated as 36. The dual tension fastening system 36 preferably comprises a primary fastening system 38 and a waist closure system 40. The primary fastening system 38 preferably comprises a pair of securement members 42 and a landing member 44. The waist closure system 40 is shown in Figure 1 to preferably comprise a pair of first attachment components 46 and a second attachment component 48. The diaper 20 also preferably comprises a positioning patch 50 located subjacent each first attachment component 46.

The diaper 20 is shown in Figure 1 to have an outer surface 52 (facing the viewer in Figure 1), an inner surface 54 opposed to the outer surface 52, a first waist region 56, a second waist region 58 opposed to the first waist region 56, and a periphery 60 which is defined by the outer edges of the diaper 20 in which the longitudinal edges are designated 62 and the end edges are designated 64. The inner surface 54 of the diaper 20 comprises that portion of the diaper 20 which is positioned adjacent to the wearer's body during use (i.e. the inner surface 54 generally is formed by at least a portion of the topsheet 24 and other components joined to the topsheet 24). The outer surface 52 comprises that portion of the diaper 20 which is positioned away from the wearer's body (i.e. the outer surface 52 generally is formed by at least a portion of the backsheet 26 and other components joined to the backsheet 26). The first waist region 56 and the second waist region 58 extend, respectively, from the end edges 64 of the periphery 60 to the lateral centreline 66 of the diaper 20. The waist regions each comprise a central region 68 and a pair of side panels which typically comprise the outer lateral portions of the waist regions. The side panels positioned in the first waist region 56 are designated 70 while the side panels in the second waist region 58 are designated 72. While it is not necessary that the pairs of side panels or each side panel be identical, they are preferably mirror images one of the other. The side panels 72 positioned in the second waist region 58 can be elastically extensible in the lateral direction (i.e. elasticised side panels 30). (The lateral direction (x direction or width) is defined as the direction parallel to the lateral centreline 66 of the diaper 20; the longitudinal direction (y direction or length) being defined as the direction parallel to the longitudinal centreline 67; and the axial direction (Z direction or thickness) being defined as the direction extending through the thickness of the diaper 20).

Figure 1 shows a specific execution of the diaper 20 in which the topsheet 24 and the backsheet 26 are unitary across the core and the chassis region and have length and width dimensions generally larger than those of the absorbent core 28. The topsheet 24 and the backsheet 26 extend beyond the edges of the absorbent core 28 to thereby form the periphery 60 of the diaper 20. The periphery 60 defines the outer perimeter or, in other words, the edges of the diaper 20. The periphery 60 comprises the longitudinal edges 62 and the end edges 64.

While each elasticised leg cuff 32 may be configured so as to be similar to any of the leg bands, side flaps, barrier cuffs, or elastic cuffs described above, it is preferred that each elasticised leg cuff 32 comprises at least an inner barrier cuff 84 comprising a barrier flap 85 and a spacing elastic member 86 such as described in the above-referenced U.S. Patent 4,909,803. In a preferred embodiment, the elasticised leg cuff 32 additionally comprises an elastic gasketing cuff 104 with one or more elastic strands 105, positioned outboard of the barrier cuff 84 such as described in the above-references U.S. Patent 4,695,278.

The diaper 20 may further comprise an elastic waist feature 34 that provides improved fit and containment. The elastic waist feature 34 at least extends longitudinally outwardly from at least one of the waist edges 83 of the absorbent core 28 in at least the central region 68 and generally forms at least a portion of the end edge 64 of the diaper 20. Thus, the elastic waist feature 34 comprises that portion of the diaper at least extending from the waist edge 83 of the absorbent core 28 to the end edge 64 of the diaper 20 and is intended to be placed adjacent the wearer's waist. Disposable diapers are generally constructed so as to have two elastic waist features, one positioned in the first waist region and one positioned in the second waist region.

The elasticised waist band 35 of the elastic waist feature 34 may comprise a portion of the topsheet 24, a portion of the backsheet 26 that has preferably been mechanically stretched and a bi-laminate material comprising an elastomeric member 76 positioned between the topsheet 24 and backsheet 26 and resilient member 77 positioned between backsheet 26 and elastomeric member 76.

This as well as other components of the diaper are given in more detail in WO 93/16669 which is incorporated herein by reference.

The absorbent core should be generally compressible, conformable, non-irritating to the wearers skin, and capable of absorbing and retaining liquids such as urine and other certain body exudates. The absorbent core might comprise a wide variety of liquid-absorbent or liquid handling materials commonly used in disposable diapers and other absorbent articles such as - but not limited to - comminuted wood pulp which is generally referred to as airfelt; meltblown polymers including coform; chemically stiffened, modified or cross-linked cellulosic fibres; tissue including tissue wraps and tissue laminates.

Examples for absorbent structures are described in U.S. Patent 4,610,678 entitled "High-Density Absorbent Structures" issued to Weisman et al. on September 9, 1986; U.S. Patent 4,673,402 entitled "Absorbent Articles With Dual-Layered Cores" issued to Weisman et al. on June 16, 1987; U.S. Patent 4,888,231 entitled "Absorbent Core Having A Dusting Layer" issued to Angstadt on December 19, 1989; EP-A-0 640 330 of Bewick-Sonntag et al.; U.S. 5 180 622 (Berg et al.); U.S. 5 102 597 (Roe et al.); U.S. 5 387 207 (LaVon).

The absorbent core can be a unitary core structure, or it can be a combination of several absorbent structures, which in turn can consist of one or more sub-structures. Each of the structures or sub-structures can have an essentially two-dimensional extension (i.e. be a layer) or a three-dimensional shape.

The absorbent core for the present invention can comprise fibrous materials to form fibrous web or fibrous matrices. Fibres useful in the present invention include those that are naturally occurring fibres (modified or unmodified), as well as synthetically made fibres, such as polyolefins, as polyethylene and polypropylene.

These fibrous materials may be used in an individualised form when the absorbent article is being produced, and an airlaid fibrous structure is formed on the line. Said fibres may also be used as a preformed fibrous web or tissue. These structures are then delivered to the production of the article essentially in endless or very long form (e.g. on a roll, spool) and will then be cut to the appropriate size. This can be done on each of such materials individually before these are combined with other materials to form the absorbent core, of when the core itself is cut and said materials are co-extensive with the core. There is a wide variety of making such webs or tissues, and such processes are very well known in the art.

In addition or alternatively to fibrous webs, the absorbent cores may comprise other porous materials, such as foams. Preferred foams are open-celled absorbent polymeric foam materials as being derived by polymerizing a High Internal Phase Water-in-Oil Emulsion (hereafter referred to as HIPE). Such polymeric foams may be formed to provide the requisite storage properties, as well as the requisite distribution properties, such as described in U.S. Patent Application Serial No. 08/563,866 (DesMarais et al.), filed November 25, 1995, copending U.S. Patent Application Serial No. 08/542,497, filed October 13, 1995 (Dyer et al.); U.S. Patent 5,387,207 (Dyer et al.), issued February 7, 1995; and U.S. Patent 5,260,345 (DesMarais et a.), issued November 9, 1993;

Optionally, and often preferably, the absorbent structures according to the present invention can comprise Superabsorbent polymers, or hydrogels. The hydrogel-forming absorbent polymers useful in the present invention include a variety of substantially water-insoluble, but water-swellable polymers capable of absorbing large quantities of liquids. Such polymer materials are also commonly referred to as "hydrocolloids", or "superabsorbent" materials. These hydrogel-forming absorbent polymers preferably have a multiplicity of anionic, functional groups, such as sulfonic acid, and more typically carboxy groups.

Most preferred polymer materials for use in making hydrogel-forming particles are slightly network crosslinked polymers of partially neutralised polyacrylic acids and starch derivatives thereof. Most preferably, the hydrogel-forming particles comprise from about 50% to about 95%, preferably about 75%, neutralised, slightly network crosslinked, polyacrylic acid (i.e. poly sodium acrylate/acrylic acid).

### Breathable Backsheet Materials

An essential element of the present invention are materials which are permeable for gases, such as air, or for vapour, such as water vapour. Permeability can be assessed by the Moisture Vapour Transmission Rate (MVTR), expressed in units of [g/24h/m2] under various driving transport forces. For the context of the present invention, the method as laid out below relates to Calcium-Chloride adsorbing moisture through the test specimen under an outside relative humidity of 75 % at 40°C.

Conventional examples for such materials are so called microporous films, as can be provided by Mitsui Toatsu Co., Japan under the designation ESPOIR NO. Such films can be made by producing a polymer film such as made from Polyethylene, further comprising filler particles, such as Calcium-Carbonate. After having formed a film wherein these filler particles are embedded into a matrix of polymeric material, the film is mechanically treated so as to strain and stretch the polymeric materials permanently, thereby creating small cracks around the non-deforming filler particles. The cracks are sufficiently small to allow gas molecules of the gas phase to pass through, but prevent liquids from penetrating.

Preferred executions of such films are currently produced by a number of film making companies, specific examples are supplied by MITSUI TOATSU, Japan, under the designation ESPOIRE NO, having - at a basis weight of about 30 gsm an MVTR result of about 3800 g/24hr/m2. An even higher breathability is exhibited by a film supplied by EXXON Chemical Co, Ill, U.S., under the designation EXXAIRE, having at a basis weight of about 30 gsm an MVTR result of about 4500 g/24hr/m2.

Within the context of the present description, the various degrees of breathability can be classified as follows:

**Table 1**

| range of permeability | MVTR [g/m2/24h] |
|---|---|
| non-permeable | up to about 200 |
| low permeability | up to about 2000 |
| medium permeability | up to about 4000 |
| high permeability | up to about 6000 |
| very high permeability | more than about 6000. |

These values should be compared to a value of about 12 000 g/m2/24h which would be required for covering human skin without providing a significant additional resistance to the moisture transfer away from the skin, or alternatively result when operating the MVTR test without a test material.

In order to improve the preferred clothlike feel of such films, these can be and are often combined with fibrous webs, such as non-wovens, which will be positioned towards the outer side of the article, i.e. on the garment side. Such fibrous webs can be non-woven materials, such as - but not limited to - polypropylene, polyethylene carded or spunbonded webs, thermobonded or chemically bonded webs. As such webs are often more expensive than the film materials, it will be desired to apply these webs only where they are required to provide the fabric- and cloth-like feel, especially in the region essentially co-extending with the core region.

Preferred non-woven materials are supplied e.g. by SANDLER GmbH, Schwarzenbach, FRG, under the designation VP39522, which is a 27 gsm carded, heat bonded polypropylene web.

The non-woven can be combined with the film materials by conventional laminating steps, like adhesive lamination using spray gluing applicators, or gravure applicators, slot die applicators and the like. Or, the materials can be combined by melting at least one component of at least one preferably both of the two materials to be laminated, or by thermal or heat lamination, dynamic, or ultrasonic laminators, or extrusion lamination.

A key element of the present invention is to combine the film and the fibrous material into a composite before the film is "activated" to become breathable.

This deformation can be achieved by a number of different ways, in machine direction of the material such as by conventional stretching between two nip roll arrangements running at a differential speed, or in CD directions such as tentering fixing the edges of the material in diverging frames, or by running it through narrowly intermeshing rolls, or by any combination thereof. Each of these steps can be executed whilst the material is heated (i.e. at a temperature exceeding the ambient temperature, i.e. most often at a temperature of more than about 40°C), or "cold", i.e. below said temperature.

A particularly suitable process according to the present invention is to treat composites comprising laminated and non-laminated zones materials as discussed above by feeding the starting material - referred to as web in the following - through at least two rolls each with circumferential ridges and grooves, which are run such that the ridges and grooves are "engaging" as described herein at such a close tolerance that the web undergoes permanent deformation, thereby providing mechanical treatment of the web.

Similar processes have been developed for treating stretch laminate materials and are described in U.S. 5.167.897 (Weber) relating to stretch materials.

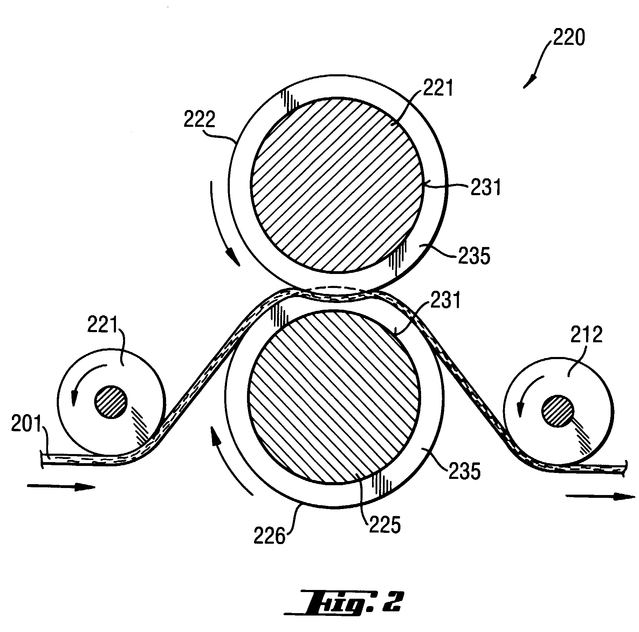

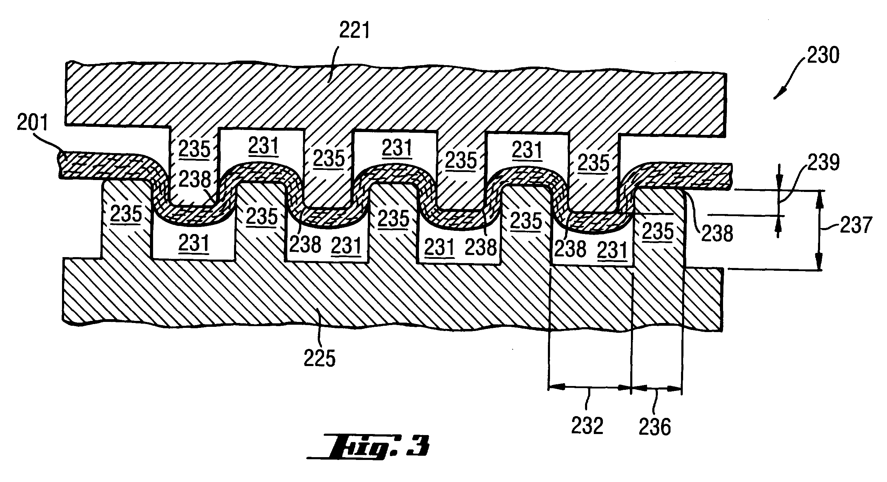

Referring to Figures 2 and 3, the essentially untensioned starting material 201 is directed through an incremental cross-directional web stretching system 220 employing opposed pressure applicators having three dimensional surfaces which at least to a degree are complementary to one another. In a preferred execution, the web 201 is directed by idler rolls 211 and 212 to pass between the ridges and grooves of the surface 222 an uppermost corrugated roll 221 and respective intermeshing grooves and ridges of the surface 226 of the lowermost corrugated roll 225. While the exact configuration, geometry, spacing and depth of the complementary ridges and grooves on the uppermost and lowermost corrugated rolls will vary, depending upon such factors as the amount of tensioning desired in the web and properties of the web itself, such as basis weight and resiliency, a specific preferred execution has an essentially rectangular profile as schematically indicated in figure 2, whereby - such as for treatment of a web having a basis weight of about 25 gsm film and 27 gsm non-woven - the grooves 231 have a width 232 of 1 mm, the ridges 235 have a width 36 of 0.6 mm, a distance 237 from bottom of the grooves to peaks of the ridges of about 8 mm, and - both at edges and corners and indicated by 238 - smoothed radii of about 0.1 mm. Also indicated in figure 3 is the cross-section of the web 201.

For webs having different properties, these dimensions should be adopted appropriately, whereby it has been found advantageous to have the width 236 of the ridges 235 to be between 30% and 90% of the width 232 of the grooves 231, preferably between 50% and 70%.

The degree of overlap 239 of the opposing peaks on the corrugated rolls may of course be adjusted, as desired, to produce more or less strong mechanical treatment in the web.

The maximum overlap 239 is dictated by operational constraints, such as running such an equipment effectively, and by material properties, which might result in perforation or cutting of the web at too strong treatment.

The minimum overlap 239 is defined by the limitation, that the web is actually mechanically treated. This requires, that the distance of the corrugated rolls, which are arranged such that they could intermesh if the distance would be narrowed, is less than the caliper of the web. Then, the "overlap" as used herein, is smaller than zero, and the lower limit of the overlap to be useful for the current invention is about -100%. Preferably, however, the overlap should be significantly more than -15% and for particularly preferred executions amount to several hundred percent or even more than 1000%.

The materials of the corrugated rolls 221 and 225 can be any suitable material to allow appropriate shaping and to withstand the pressure which the rolls exert to the material, such as metal like aluminum alloys or steel. In case of too low or too high friction between the web and the rolls, the roll surface may be roughened or smoothed or otherwise treated to prevent the web to slip between the corrugation or to not penetrate sufficiently into the corrugations.

It is also recognised that whilst a preferred execution comprises a pair of meshing corrugated rolls having their corrugations aligned substantially parallel to one another, the present invention may also be practiced by employing pairs of corrugated rolls wherein the corrugations are not all oriented parallel to one another. Furthermore, the corrugation on such pairs of corrugated rolls need not necessarily be aligned parallel to either the machine or the cross machine direction.

Whilst this has been described by referring to one preferred profile 230, other profiles can be used. The grooves and ridges can be in triangular, trapezoidal shape, or more rounded, e.g. in sinusoidal shape, or any other shape allowing intermeshing of two rolls. Obviously, for such arrangements, other preferred dimensions can be readily elaborated for optimal performance.

Whilst a currently preferred execution has uniform arrangement of the ridges and groove both in circumferential and axial direction of the corrugated rolls, specific executions can comprise regions with different patterns, be this in an axial arrangement, e.g. widths of grooves and/or ridges changing across the axial direction of the rolls, or be this in circumferential direction, e.g. the ridges and grooves have a changing depth across the circumference of at least one roll, or at least one of the rolls has an macroscopically curvatured shape, e.g. is thicker in the centre portion than towards the edges. These applications then induce either MD-oriented stripes or zones of breathability, and/or CD oriented regions with different degrees of breathability.

Also, the use of more than two corrugated rolls can be beneficial, such as when to avoid too strong treatment in one step. Thereby, a further roll of the characteristics of roll 225 can be positioned such that its corrugations also intermesh with the corrugations of roll 221. Whilst the corrugations of this roll should be aligned with the ones of the roll 225, the depth of intermeshing can be different or - within the limitations of the alignment - the shape of the corrugations can be different, such as having different radii of the ridges and grooves. Of course, also having more than two rolls can be contemplated, or two sets of pairs of rolls 221 and 225, or other combinations of such set-ups.

In addition to the described treatment, the web may further be longitudinally stretched in machine direction, either in a separate process step before or after the cross directional stretching or integrated into the process thereof.

A further enhancement of the process can be achieved by further adding a process step of heating the web, either by a separate process step directly after the post formation treatment as disclosed in the above, or by heating the means that applies the mechanical stress to the web, e.g. one or both of the corrugated rolls. Preferentially, this is applied for webs comprising thermofusible materials (such as the materials comprising thermoplastic fibres). The beneficial effect of this additional heat treatment lies in that the webs can be formed such as to allow relatively easy plastic deformation by the mechanical process, and reaching a desired resiliency, stability, strength or uniformity by the heat curing.

It is further recognised that while the preferred processes herein disclosed employ meshing cylindrical corrugated rolls, the present invention may also be carried out utilising an intermittent stamping operation employing meshing plates to incrementally stretch the web in question.

By this treatment, breathability will be imparted to the composite, to different degree for various regions or zones thereof. In particular, the region of the film non-woven laminate will have less activation energy available for the deformation of the film, as also the fibrous layer will be deformed.

Thus, materials useful for the present invention can be made by starting from the same basis as conventional breathable films are made from, for example calcium-carbonate filled materials, such as described in PCT Application WO 97/1544, or as made as precursor for the above mentioned films.

However, in contrast to these conventional films, the ones for being used in absorbent articles according to the present invention are not activated to be subsequently optionally combined with a non-woven, but are first combined to be then activated.

A specific example, a calcium-carbonate film polyethylene film as available from Clopay Plastic Product Company Inc, Cincinnati, OH, U.S., under the designation 9704250 combined while the freshly extruded film material is still above its softening point temperature so as to be easily heat bonded with a 23 gsm spunbonded bi-component web having polyethylene sheath and a polypropylene core such as supplied by SANSEKI Co., Japan. The width of the film can be a typical width of a baby diaper, such as about 330 mm, the width of the non-woven can be about 150 mm, positioned centred on the film.

Treating this film in a manner as described in the above will then result in MVTR values of about 1700 g/24hr/m2 in the zone of the film/non-woven laminate, and of about 2100 in the zones having film without non-woven laminate.

If the fibrous layer is applied in a stripwise fashion, and in even activation conditions are chosen across the width of the activator, the different amount of materials as run through the essentially same process settings, will result in imparting higher breathability in the zones having no non-woven. In view of the compatibility with designs as described below, the different zones should have a difference of at least 20% when being related to the lower value. In absolute terms, the difference is preferably more than 500 g/m2/24h. For example, the treatment can result in an increased MVTR result, for example having 1000 g/24hr/m2 in the laminate zone, and more than about 1600 g/24hr/m2 in the other zones, or 1500 g/24hr/m2 in the laminated zone and 2200 in the other zone.

In case the fibrous and the film layer are essentially coextensive, a different degree of breathability can be achieved by different treatment conditions in different zones, such as having about twice the amount of activating teeth in one zone as compared to the other. These zones can also represent regions with continuously changing activation and hence breathability values instead of step arise changes.

In a further embodiment, the positioning of the fibrous layer in zones or stripes can be combined with the difference in treatment.

In a further aspect of the present invention, the films respectively laminates as treated in the above, may be further combined with other materials which should not negatively impact the breathability effects as imparted by the treatment, such as with other non-woven materials, which additionally provide benefits for improved softness feel.

### Regions of the article

However, apart from the selection of the appropriate materials, the arrangements of the materials within the article are of high importance. For the scope of the following description, the article is being considered to consist essentially of two regions, namely one part of the article comprising the absorbent core (generally in the central region of the article), the other part complementing the rest of the article.

Thus, the "core region" covers the regions which will in use cover the body opening from which the exudates are discharged, and will further extend up to into the waist region, or regions.

Apart from liquid handling means and auxiliary means such as elements to maintain the various other elements together (e.g. adhesives), this core region will comprise one or more materials which are intended to face towards the skin of the wearer during use, and which are generally referred to as topsheet materials, and one or more materials which are intended to cover the opposite surface of the article (i.e. the outside), thus for example aiming to be oriented towards the clothes of the wearer.

The "chassis region" comprises the design elements of the article to hold the article on the wearer (i.e. fixation means), the elements to prevent the exudates from leaking out of the article (e.g. the leg closure elastication means, or the waist features), and means to connect the various elements.

Also the chassis region will comprise one or more material which is intended to face towards the skin of the wearer during use, and which is generally referred to as topsheet, and one or more materials which are intended to cover the opposite surface of the article (i.e. the outside), thus for example aiming to be oriented towards the clothes of the wearer, which are generally referred to as backsheet.

With regard to fluid permeation properties, i.e. gas permeability and liquid impermeability, there can be different requirements for the backsheet materials in the chassis and core region of the article.

In the chassis area, the backsheet material should allow prevention of occlusion of the skin and thus allow vapour to evaporate through very easily, i.e. a high gas permeability, but the material does not need to satisfy specific requirements for liquid impermeability.

In the core area, there is an additional requirement for the backsheet material to better retain free liquid, such as before this is absorbed, or when the absorbent structure reaches saturation.

Within the definition of materials covering certain regions of the article, it should be noted, that a core covering backsheet material may extend somewhat into the chassis region. For example, if the core covering material is made from an endless roll, it can very well be, that the core covering region material is made from a strip-like material of the width of the core, either exactly or somewhat wider in case of a rectangular core, or covering the most critical zones of the core if not rectangular shape, and this strip can extend into the end-flap region of the article, i.e. along the full length of the article.

### Test Procedures

### Moisture Vapour Transmission Rate

The Moisture Vapour Transmission Rate is measuring the amount of moisture adsorbed by calcium-chloride in a "cup" like container covered with the test specimen from controlled outside air conditions (40 ± 3°C/ 75 ± 3% relative humidity).

The sample holding a cup is a cylinder with an inner diameter of 30 mm and an inside height from bottom to top flange of 49 mm. A flange having a circular opening to match the opening of the cylinder can be fixed by screws, and a silicone rubber sealing ring, matching the inner diameter, fits between the top flange and the cylinder. The test specimen is to be positioned such that it covers the cylinder opening, and can be tightly fixed between the silicone rubber sealing and the upper flange of the cylinder.

The equipment as well as the test specimen should be well adjusted to the temperatures, and the constant temperature/humidity chamber preferably has a size to accommodate up to 30 samples.

The absorbent desiccant material is calcium-chloride, such as can be purchased from Wako Pure Chemical Industries Ltd., Richmond, VA, U.S. under the product designation 030-00525. If kept in a sealed bottle, it can be used directly. It also can be sieved to remove lumps, or excessive amounts of fines, if existing. It also can be dried at 200°C for about 4 hrs.

15.0 ± 0.02 g of calcium-chloride are weighed into the cup, and tapped lightly so as to level it out, such that the surface is about 1 cm from the top of the cup.

The samples, which are cut to about 3.2 cm by 6.25 cm, are placed flat and overlapping with the seal over the opening, and the seal and the top flange are affixed by the screws without over-tightening. The total weight of the cup assembly is accurately recorded on a four decimal places scale, and the assembly is placed into the constant temperature/humidity chamber.

After 5 hrs (without opening of the chamber), the sample is removed and immediately covered tightly with non-vapour permeable plastic film such as Saran wrap as commonly used in the U.S. After about 30 mins to allow for temperature equilibration, the plastic film cover is removed and the accurate weight of the assembly is recorded.

The MVTR value is then calculated from the moisture increase during these 5 hours through the 3 cm circular opening and then converted to units of "g/24h/m2".

For each test, three replicates should be run, the resulting values will be averaged, and the result rounded to the nearest 100 value.

Overall, this method is applicable to thin films, multi layer laminates and the like. Experience has shown, that typical standard deviations range between 50 and 250 g/24hr/m2 for averaged values of up to about 5000 g/24hr/m2.

Due to this range, materials being considered to be essentially vapour impermeable such as conventional PE films, are reported as having a MVTR of about 200 g/24hr/m2.

If the units for an MVTR value are omitted for simplicity, a material "having a MVTR value of 1000" should accurately be a material "having a MVTR value of 1000 g/24h/m2" according to this method.

## Claims

1. Absorbent article comprising
an absorbent core defining a core region;
a chassis region surrounding said core region;
said absorbent core being interposed between a backsheet, which is impermeable to liquids, and a topsheet, which is permeable to liquids;
said backsheet extending over the core region and at least parts of the chassis region,
said backsheet further comprising a fibrous layer at least in the core region;
characterised in that
the backsheet comprises film or film-like layer which is permeable to vapours or gases which is a unitary layer extending both into the core region and at least into parts of the chassis region,
whereby the backsheet has a higher MVTR value in these parts of the chassis region, which comprise said film or film-like layer, than the backsheet in the core region, which comprise said film or film-like layer and the fibrous layer.

2. An absorbent article according to claim 1, wherein said film or film-like layer is wider than said fibrous layer.

3. Absorbent article according to claim 1 or 2
further characterised in that
the backsheet material in the core region has an MVTR of at least 500 g/24hr/m2.

4. Absorbent article according to claim 3
further characterised in that
the backsheet material in the core region has a MVTR of at least 1500 g/24hr/m2.

5. Absorbent article according to any of claims 1 to 4
further characterised in that
the MVTR values of the backsheet in the chassis region are at least 20% higher than the MVTR values of the backsheet in the core region.

6. Absorbent article according to any of claims 1 to 4
further characterised in that
the MVTR values of the backsheet in the chassis region are at least 500 g/24hr/m2 higher than the MVTR values of the backsheet in the core region.

7. Absorbent article according to any of the preceding claims, whereby
said film or film-like layer comprises a polymeric matrix and particulate filler material embedded in said matrix.

8. An absorbent article according to claim 7, whereby
the filler material is calcium carbonate.

9. An absorbent article according to any of the preceding claims, whereby said film or film-like layer in the chassis region has a basis weight of less than 50 gsm.

10. An absorbent article according to any of the preceding claims, whereby said backsheet has a basis weight of less than 70 gsm where it comprises said film or film-like layer and said fibrous layer.

11. An absorbent article according to any of the preceding claims, whereby said fibrous layer is a non-woven web.

12. An absorbent article according to any of the preceding claims, whereby the film or film-like layer and the fibrous layer are combined by heat or melt bonding.

13. An absorbent article according to any of the preceding claims, whereby the film or film-like layer and the fibrous layer are combined by extrusion coating.

14. An absorbent article according to any of the preceding claims, whereby the film or film-like layer and the fibrous layer are combined by adhesive.

15. Absorbent article according to any of the preceding claims, whereby the article is a baby diaper or an adult incontinence article.

16. A process for inducing zoned vapour or gas permeability into a laminate for being used as a backsheet in a product according to any of the preceding claims, comprising the steps of
- providing a polymeric film comprising particulate filler embedded in the polymeric matrix;
- providing a fibrous web which has equal width or is narrower than the film in cross-machine direction;
- combining the film and the web to form a laminate;
- stretching the laminated and the non-laminated film zones by feeding the film and laminate zones between a pair of opposed pressure applicators comprising three-dimensional surfaces which are complementary to one another; and
- subjecting the portions of said web located between said opposed pressure applicators to incremental cross dimensional elongation by causing said opposed three-dimensional surfaces of said pressure applicators mesh with one another,
whereby said laminated and non-laminated film zones are at least partially permanently deformed and different vapour gas permeability is induced in various zones thereof.

17. A process according to claim 16 whereby the fibrous web is narrower than the polymeric film in CD direction.

18. A process according to claim 16 or 17, further comprising the step of heat treating the web after having subjected the web to said incremental CD elongation step.

19. A process according to any of claims 16 to18, whereby the intermeshing between the two pressure applicator rolls is essentially constant throughout the width of the laminated and non-laminated zones.

20. A process according to claim 16, whereby the intermeshing between the two pressure applicators is different throughout various zones.
